# FASCICULE DE BREVET EUROPEEN

(11) **EP 1 828 318 B1**
(45) Date de publication et mention de la délivrance du brevet: **12.11.2008**
(21) Numéro de dépôt: 05850569.4
(22) Date de dépôt: 21.12.2005
(51) Int. Cl.: C09C 1/62, C09C 1/66, C09C 3/08, C09C 3/00

(54) **COMPOSITION DE PIGMENTS METALLIQUES**
METALLPIGMENTZUSAMMENSETZUNG
METAL PIGMENT COMPOSITION

(30) Priorité: 22.12.2004 FR 0413727
(43) Date de publication de la demande: 05.09.2007
(73) Titulaire: Toyal Europe, 64490 Accous (FR)
(72) Inventeur: MORVAN, Fabrice, F-64400 Oloron Sainte Marie (FR)
(74) Mandataire: Sueur, Yvette
(86) Numéro de dépôt international: PCT/FR2005/003224
(87) Numéro de publication internationale: WO 2006/070108

(56) Documents cités:
- EP-A- 0 326 737
- EP-A- 0 936 253
- DE-A1- 19 515 538

## Description

La présente invention concerne une composition de pigments métalliques, notamment de pigments d'aluminium, pour la préparation de peintures métallisées.

La technique utilisée classiquement dans l'industrie pour la préparation de compositions de pigments métalliques destinées à la préparation de peintures métallisées consiste à introduire le métal sous forme de particules dans du white spirit, et de soumettre la suspension à un broyage en présence d'un agent lubrifiant, du type acide gras. Cette technique est décrite notamment dans US-2 002 891. Cette technique permet d'obtenir des pigments ayant de bonnes propriétés. Cependant, elle présente un inconvénient majeur en raison de la grande volatilité et de la toxicité du white spirit, qui en outre n'est pas biodégradable.

EP-0 936 253 décrit un procédé de préparation de composition de particules métalliques consistant à broyer des particules du métal dans un milieu constitué par le produit d'estérification d'une huile végétale et contenant un acide gras comme lubrifiant. Le milieu comprend essentiellement un mélange d'esters des divers acides gras qui constituent l'huile végétale utilisée comme source d'esters. Cette technique apporte des avantages par rapport à l'utilisation de white spirit, puisque les esters d'acide gras ne sont ni volatils, ni toxiques, et en outre ils sont biodégradables. Cependant, l'utilisation comme support de broyage des particules métalliques, d'un mélange d'esters d'huile végétale et d'un acide gras comme lubrifiant présente certains inconvénients. D'une part, elle favorise l'agglomération des particules lors du vieillissement de la pâte pigmentaire qui les contient, lors de son stockage. Or l'utilisation pour la fabrication d'une peinture métallisée d'une composition de particules métalliques ayant un degré d'agglomération des particules métalliques plus élevé provoque une diminution de l'aspect "métallisé" de la peinture et une augmentation de la densité de la couleur associée. En effet, l'agglomération des particules métalliques diminue la surface réfléchissante développée par les pigments qui perdent donc leur force teintante : dans une composition de vernis coloré qui contient lesdites particules, lesdits pigments dégradent par conséquent moins la couleur du vernis qui les contient. D'autre part, la présence de l'acide gras en plus des esters d'acide gras dans la composition de particules limite l'efficacité du broyage en assurant un rôle lubrifiant supplémentaire. En outre, après le broyage, la récupération du solvant support de broyage en vue de son recyclage nécessite une distillation.

Le but de la présente invention est de fournir de nouvelles compositions de pigments métalliques qui ne présentent pas les inconvénients des compositions de l'art antérieur, ainsi qu'un procédé pour leur fabrication.

Le procédé selon l'invention pour la préparation d'une composition de pigments métalliques, consiste à introduire des particules initiales du métal (Pi) dans un liquide support et à soumettre le mélange ainsi obtenu à un broyage. Il est caractérisé en ce que le liquide support est constitué par un ester d'un acide gras R'-COOR dans lequel R' est un groupe aliphatique saturé ou insaturé ayant de 9 à 20 atomes de carbone, et R est un alkyle ayant de 1 à 8 atomes de carbone.

Parmi les ester R'-COOR, on préfère ceux dans lesquels R' est un groupe saturé CₙH₂ₙ₊₁, n étant compris entre 9 et 20, et plus particulièrement ceux dans lesquels 9≤n≤17. A titre d'exemple, on peut citer le caprate de méthyle, le laurate de méthyle, le myristate de méthyle, le palmitate de méthyle et le stéarate de méthyle.

Le procédé peut être mis en oeuvre pour la préparation de compositions de pigments métalliques dans lesquelles le métal est choisi parmi l'aluminium, le cuivre, le zinc, l'étain, l'or, l'argent, les alliages de ces métaux, ainsi que les aciers inoxydables et le bronze. Il est particulièrement intéressant pour la préparation de compositions de pigments d'aluminium qui sont utilisées notamment pour la préparation de peintures métallisées.

Les particules de métal Pi peuvent être sous forme de poudres quelconques, constituées par des particules essentiellement sphériques ou non, ou par des bâtonnets. Elles peuvent également être formées par des feuilles minces, telles que les feuilles d'aluminium. Dans le cas de l'aluminium, on peut utiliser en particulier des poudres constituées par des particules dont la teneur en aluminium métal est d'au moins 99% en masse, ayant une dimension moyenne de 0,1 à 500 µm. A titre d'exemple, on peut citer les particules d'aluminium commercialisées par les sociétés Toyal Europe SA, Toyal America Inc. ou Toyo Aluminium K.K. sous les dénominations "grade 406S", "grade 409S" et "grade 432.

De préférence, le mélange soumis au broyage contient de 1 à 10 kg de liquide support pour 1 kg de particules Pi.

Le broyage a pour but de déformer plastiquement les particules métalliques initiales Pi qui, d'une forme plus ou moins sphérique ou d'une forme de film, se transforment en particules Pf qui ont un facteur de forme (représentant le rapport de l'épaisseur moyenne au diamètre transversal moyen), compris entre 1/5 et 1/1000. De telles particules peuvent se présenter sous forme de paillettes, de disques ou de flocons plus ou moins réguliers. La forme et la dimension des particules Pf dépend de l'efficacité du broyage. Un broyage moins efficace donne des particules Pf régulières de plus grandes dimensions. Si le broyage est plus efficace, éventuellement dans des conditions de surbroyage, les particules en cours de formation sont cassées et donnent des particules finales Pf de dimensions plus petites, et éventuellement plus irrégulières.

La durée du broyage est généralement comprise entre 1 h et 20 h. Elle est adaptée en fonction de l'énergie développée par le dispositif de broyage, de la nature des particules Pi et du résultat souhaité. Le choix de la durée de broyage est à la portée de l'homme de métier.

A la fin de l'étape de broyage, la teneur en ester d'acide est ajustée à une valeur comprise entre 30 et 90% en masse pour former une composition constituée par des particules métalliques Pf et l'ester d'acide gras, qui est directement utilisable pour les diverses applications envisagées, et qui peut être conservée jusqu'à son utilisation. L'ajustage peut être effectué en soumettant le mélange obtenu après broyage à l'action d'un filtre presse pour éliminer l'excédent de liquide support, ou en ajoutant de l'ester d'acide gras par malaxage si la teneur est insuffisante.

Un autre objet de l'invention est une composition de pigments métalliques constituée par des particules métalliques Pf et un liquide, caractérisée en ce que le liquide est un ester d'un acide gras R'-COOR dans lequel R' est un groupe aliphatique saturé ou insaturé ayant de 9 à 20 atomes de carbone, et R est un alkyle ayant de 1 à 8 atomes de carbone.

Les compositions contenant un ester dont le substituant R est un groupement méthyle et le substituant R' est un groupement alkyle saturé ayant de 9 à 17 atomes de carbone sont particulièrement préférées.

La teneur en ester d'une composition selon l'invention est comprise de préférence entre 30 et 90% en masse.

Les particules métalliques Pf sont des particules anisotropes ayant des dimensions moyennes inférieures ou égales à 500 µm, et un facteur de forme compris entre 1/5 et 1/1000. De préférence, les particules Pf sont du type paillettes, avec un diamètre transversal moyen inférieur ou égal à 500 µm et une épaisseur moyenne inférieure ou égale à 3 µm.

Une composition de pigments métalliques selon la présente invention peut être utilisée dans divers domaines techniques. Par exemple, elle peut être utilisée pour la formulation d'une peinture métallisée destinée notamment à l'industrie automobile, ou pour la formulation de peintures industrielles. Une composition de pigments métalliques selon l'invention peut en outre être utilisée pour la formulation d'une encre d'impression ou d'une matière plastique à aspect métallisé. En outre, une composition de pigments selon l'invention peut être utilisée pour l'élaboration d'une composition cosmétique à aspect métallisé.

Lorsqu'une composition de pigment selon l'invention est utilisée pour l'élaboration d'une peinture métallisée, la forme et la dimension des particules Pf a une influence sur l'aspect de la peinture finale. De manière générale, toutes choses étant égales par ailleurs :
- une augmentation du diamètre transversal moyen des particules induit une augmentation de la brillance d'une peinture à laquelle les particules sont intégrées ;
- une diminution du diamètre transversal moyen provoque un pouvoir couvrant plus élevé qui dégrade davantage la couleur associée à la peinture ;
- l'augmentation de la régularité des particules augmente la brillance de la peinture qui les contient, alors qu'une forte irrégularité donne des peintures ternes ;
- une augmentation de la proportion des particules favorise l'aspect métallisé de la peinture.

La présente invention est décrite plus en détail à l'aide des exemples suivants, auxquels elle n'est cependant pas limitée.

Les produits utilisés sont les suivants :
- Poudre d'aluminium conventionnelle, non sphérique, désignée ci-après par Al-05 (d₅₀ = 5 µm, Al > 99,7%) ;
- Poudre d'aluminium conventionnelle, non sphérique, désignée ci-après par Al-10 (d₅₀ = 10 µm, Al > 99,7%) ;
- laurate de méthyle commercialisé sous la dénomination Radia 7118 par la société Oléon ;
- Vernis nitrocellulosique incolore, commercialisé par la société Nouvion sous la dénomination NS813 ;
- Vernis nitrocellulosique bleu, obtenu par mélange de vernis nitrocellulosique NS813 et de bleu de phtalocyanine ;
- Mélange d'acides gras à forte teneur en acide oléique (90%) commercialisé par la société Oléon sous la dénomination Radiacid 294.

Dans les divers exemples,
- le broyage a été effectué dans un broyeur cylindrique ayant un diamètre de 50 mm et une profondeur interne de 200 mm, et contenant 39,2 kg de billes d'acier ayant un diamètre inférieur à 10 mm ;
- la préparation des vernis est effectuée dans un mélangeur présentant un mouvement de type planétaire, commercialisé par la société Kurabo sous la dénomination MS NSB 350N.

### Exemple 1

### Préparation de compositions de pigments d'aluminium

On a introduit dans un broyeur, une quantité n_{A} de poudre d'aluminium Pi, une quantité n_{L} de laurate de méthyle, et l'on a soumis au broyage pendant une durée de 8 heures à 28 t/min.

A l'issue du broyage, on a obtenu une boue qui a été tamisée sur des tamis d'ouverture 25 µm, puis filtrée sur un filtre à plaque. La pâte obtenue après filtration a été homogénéisée à l'aide d'un malaxeur à pales du type sigma, avec addition d'ester pour ajuster le taux d'ester à 65%.

L'essai a été répété en modifiant la nature et la quantité de la poudre d'aluminium Pi, et la quantité d'ester, pour obtenir les échantillons BR03 et BR06, selon le tableau 1.

**Tableau 1**

| Echantillon | Pi | n_{A} (g) | n_{L} (g) |
|---|---|---|---|
| BR03 | Al-05 | 802 | 2350 |
| BR06 | Al-10 | 892 | 6200 |

On a reproduit le mode opératoire particulier de l'échantillon BR06, pour préparer les échantillons BR07 à BR011, mais en utilisant pour BR07, le liquide récupéré après l'étape de tamisage-filtrage de l'échantillon BR06, et en utilisant ensuite, pour la préparation de chacun des échantillons BRn (n = 8 à 11), le liquide récupéré après l'étape de tamisage-filtrage de l'échantillon BR(n-1).

A titre comparatif, on a reproduit le mode opératoire de l'exemple 1, mais en utilisant comme liquide support de broyage, d'une part un mélange de white spirit (n_{W}, en g) et de Radiacid (n_{R}, en g) (Ech. BR01-C, BR05-C) et d'autre part un mélange de laurate d'éthyle (n_{L}, en g) et de Radiacid (n_{R}, en g) (Ech. BR02-C). Les conditions particulières sont indiquées dans le tableau 2 suivant.

**Tableau 2**

| Ech. | Pi | n_{A} (g) | n_{W} (g) | N_{R} (g) | N_{L} (g) |
|---|---|---|---|---|---|
| BR01-C | Al-05 | 802 | 2350 | 124 | - |
| BR02-C | Al-05 | 802 | - | 124 | 2350 |

La granulométrie avant tamisage de divers échantillons a été déterminée. On a dispersé 0,32 g d'un échantillon dans 8 mL de butyl glycol, puis on a redispersé le mélange obtenu dans 40 mL d'éthanol. Après homogénéisation à la spatule, on a soumis le mélange à des ultra-sons (60 Hz) pendant 3 min. Le mélange a ensuite été caractérisé à l'aide d'un granulomètre de type Malvern Mastersizer 2000, équipé du module de mesure en voie liquide Hydro 2000 S.

Les résultats sont indiqués dans le tableau 3 suivant.

**Tableau 3**

| Ech. | d₁₀ (µm) | d₅₀ (µm) | d₉₀ (µm) |
|---|---|---|---|
| BR01-C | 7,9 | 15,7 | 28,4 |
| BR03 | 6,2 | 12,8 | 24,0 |
| BR02-C | 10,2 | 19,5 | 34,3 |
| BR06 | 15,0 | 24,9 | 40,9 |
| BR07 | 14,8 | 24,4 | 39,7 |
| BR08 | 14,2 | 23,4 | 38,2 |
| BR09 | 15,1 | 24,9 | 40,5 |
| BR10 | 15,5 | 25,5 | 41,6 |
| BR11 | 14,0 | 23,0 | 37,4 |

La comparaison des résultats pour les échantillons BR06 à BR11 selon l'invention montrent que l'utilisation du liquide support de broyage recyclé ne dégrade pas la granulométrie des pigments.

La comparaison des résultats obtenus pour BR01-C et pour BR03 montre que la répartition des dimensions de particules est sensiblement équivalente lorsque le mélange "white spirit+Radiacid" de l'art antérieur (Ech. BR01-C) est remplacé par l'ester selon l'invention (Ech. BR03).

La comparaison des valeurs d₅₀ obtenues pour BR03 et pour BR02-C indique des particules plus grosses lorsque le liquide support de broyage est un mélange de laurate de méthyle et Radiacid. Il se confirme ainsi que, lorsque le liquide de support de broyage est un ester d'acide gras, la présence d'acide gras diminue l'efficacité du broyage, auquel cas les particules subissent une déformation les faisant croître sans subir de rupture. Le broyage se fait alors sans surbroyage, c'est-à-dire sans génération de paillettes de faible granulométrie obtenues par rupture de paillettes de plus grande taille.

### Exemple 2

### Evaluation du pouvoir couvrant des pigments

Une composition de vernis est préparée de la manière suivante. On disperse 1,5 g d'un échantillon de composition de pigments dans 48,5 g de vernis nitrocellulosique bleu, et on homogénéise le mélange dans un mélangeur. Le vernis coloré ainsi obtenu est appliqué sur une feuille de papier.

Un vernis bleu a ainsi été préparé d'une part avec la composition de pigments selon l'invention BR03, et d'autre part avec les compositions de pigments de l'art antérieur BR01-C et BR02-C.

Après application des vernis respectifs sur une feuille de papier, le pouvoir couvrant a été évalué en observant par transparence la lumière transmise à travers chacune des feuilles de papier enduites. L'on a ainsi constaté que :
- le vernis obtenu à partir de la composition de pigments BR03 à un pouvoir couvrant identique à celui du vernis obtenu à partir de la composition BR01-C, ce qui confirme que le mélange white spirit-acide gras en tant que liquide support de broyage peut avantageusement être remplacé par un ester d'acide gras ;
- le vernis obtenu à partir de BR02-C a un pouvoir couvrant moindre que le vernis obtenu à partir de BR03. Ce résultat confirme que le laurate de méthyle assure un rôle de lubrifiant lors du broyage, et que l'addition d'un acide gras donne un pouvoir lubrifiant global très élevé, qui tend à limiter l'efficacité du broyage et à donner des particules de plus grandes dimensions. La composition de vernis qui contient les pigments issus de BR02C a par conséquent un pouvoir couvrant moindre que la composition de vernis qui contient les pigments issus de BR03.

### Exemple 3

### Tests de vieillissement

On a comparé le comportement dans le temps de diverses composition de pigments selon l'invention (BR03, BR06, BR09, BR11), et de la composition de pigment BR02-C de l'art antérieur. La comparaison a été faite par un test de vieillissement accéléré, selon la procédure suivante.

Pour chaque composition de pigments, on a préparé deux échantillons que l'on a conservés respectivement à une température de 4°C, c'est-à-dire dans des conditions dans lesquelles il est admis que la composition n'évolue pas, et à 50°C, ce qui est supposé provoquer un vieillissement accéléré.

Au cours du vieillissement, on a prélevé une partie de chacun des échantillons après 3 mois, 6 mois et 9 mois, et on a préparé à partir de chaque partie prélevée, un vernis nitrocellulosique bleu selon le mode opératoire de l'exemple 2.

Il est connu que, dans une composition de pigments, les pigments ont tendance à s'agglomérer au cours du temps, et qu'une augmentation du taux d'agglomération des pigments dans une composition de pigments augmente l'intensité de la coloration d'une peinture qui contient une telle composition de pigments, au détriment de la force teintante des pigments.

L'évolution de la force teintante des pigments a été évaluée dans le système colorimétrique CIELab 1976 exprimé en coordonnées polaires [h, C, L], à l'aide du paramètre C qui renseigne sur la saturation d'une couleur. Plus le paramètre C est grand, plus la couleur est saturée et pure. Plus le paramètre C est faible, plus la couleur est dégradée et tend vers le gris. Ainsi, si l'on compare deux compositions de vernis ayant la même teinte bleue (même paramètre h), le paramètre C sera plus faible pour le vernis dans lequel les pigments seront moins agglomérés et auront une force teintante supérieure.

Les valeurs obtenues à l'aide d'un dispositif Minolta CR300 sont données dans le tableau 4 suivant. ΔC = C_{référence} -Ct, Cₜ étant la valeur mesurée après un temps de vieillissement t.

**Tableau 4**

| | ΔC (3 mois) | ΔC (6 mois) | ΔC (9 mois) |
|---|---|---|---|
| BR02-C | -0,72 | -0,62 | -0,65 |
| BR03 | -0,10 | 0,00 | -0,16 |
| BR06 | 0,01 | 0,01 | 0,04 |
| BR09 | 0,11 | 0,08 | -0,03 |
| BR11 | -0,03 | -0,11 | 0,24 |

Il apparaît ainsi que, pour l'échantillon BR03 selon l'invention, C varie très peu jusqu'à 9 mois, contrairement à ce qui est observé pour l'échantillon BR02-C selon l'art antérieur, qui contient de l'acide oléique. La simple observation visuelle de supports enduits des compositions de vernis respective confirme ce résultat. L'on constate également que pour les autres échantillons selon l'invention (BR06, BR09 et BR11), C ne varie pratiquement pas pendant les 9 mois du test de vieillissement.

### Exemple 4

L'influence des pigments sur divers paramètres d'une laque de finition brillante a été évaluée

Une composition de peinture désignée ci-après par JB 042, présentant un séchage « chimique », est préparée de la manière suivante. On a mélangé 20,0 g d'un échantillon de la composition de pigments BR03 préparé selon l'exemple 1, avec un mélange de constituants comprenant 71,67 g d'une résine alkyde-uréthane commercialisée sous la dénomination Lixothan UAL 55.55 BT, 1,45 g de siccatifs combinés commercialisés sous la dénomination Octa Soligen 161/D60, 1,2 g d'agents anti-peaux commercialisés sous la dénomination Exkin 518, et 5,68 g de white spirit. La composition de peinture ainsi obtenue a été homogénéisée à l'aide d'un mélangeur.

Un mélange de référence désigné ci-après par JB 041 a été préparé de façon similaire en remplaçant la composition de pigments BR03 par la composition de pigments de l'art antérieur BR01-C définie dans le tableau 2 ci-dessus.

### Brillance

Les peintures métallisées JB 042 et JB 041 ont été appliquées avec une épaisseur de film humide de 150 µm sur une plaque métallique. La brillance du film de peinture a été évaluée sous un angle de 60° et de 85° à différents temps d'observation. Les résultats sont indiqués dans le tableau 5 suivant. J+1, J+7 et J+30 désignent le moment de l'observation, c'est-à-dire respectivement 1 jour, 7 jours et 30 jours après le dépôt du film de peinture.

**Tableau 5**

| | J+1 | | J+7 | | J+30 | |
|---|---|---|---|---|---|---|
| | 60° | 85° | 60° | 85° | 60° | 85° |
| JB 041 | 58,5 | 66,5 | 55,7 | 65,9 | 53,2 | 64,4 |
| JB 042 | 74,5 | 91,1 | 55,5 | 83, 5 | 54,4 | 78 |

Ces résultats montrent que la brillance du film selon l'invention est nettement supérieure à celle du film selon l'art antérieur à J+1. La différence de brillance diminue au cours du temps, mais la brillance reste néanmoins supérieure pour le film selon l'invention.

### Test de vieillissement accéléré

Les peintures métallisées JB 041 et JB 042 ont été appliquées avec une épaisseur de film humide de 150 µm sur une plaque métallique. Les plaques ainsi obtenues ont été conservées à l'obscurité et leurs coordonnées colorimétriques (Système Lab) ont été mesurées à intervalles réguliers afin d'évaluer une éventuelle modification de leur couleur (jaunissement). Les résultats sont donnés dans le tableau 6 ci-dessous.

**Tableau 6**

| | J+1 | | | J+7 | | | J+14 | | | J+30 | | |
|---|---|---|---|---|---|---|---|---|---|---|---|---|
| | **L*** | **a*** | **b*** | **L*** | **a*** | **b*** | L* | **a*** | **b*** | **L*** | **a*** | **b*** |
| JB 041 | 78,12 | -0,54 | -1,73 | 78,32 | -0,55 | -1,68 | 78,58 | -0,55 | -1,58 | 78,64 | -0,65 | -1,46 |
| JB 042 | 76 | -0,55 | -1,75 | 76,86 | -0,57 | -1,7 | 76,88 | -0,57 | -1,56 | 77 | -0,67 | -1,39 |

Ces résultats montrent une évolution similaire des coordonnées colorimétriques des films de peinture, notamment le paramètre b*, dont l'évolution vers les valeurs positives indique un jaunissement du film, qui augmente de la même façon pour la peinture de référence JB 041 et pour la peinture selon l'invention JB 042.

### Séchage du film de peinture

Les peintures métallisées JB 041 et JB 042 sont appliquées avec une épaisseur de film humide de 100 µm sur une plaque de verre. Une bille métallique est maintenue sur le film de peinture humide à l'aide d'un poids de 10 g. Un mouvement de translation linéaire à vitesse constante (6 cm/h) est imposé à la bille qui se déplace ainsi linéairement à la surface du film de peinture en laissant une trace dont l'analyse révèle la nature du séchage en fonction du temps.

Typiquement, on considère que le film commence à sécher lorsqu'il ne se referme plus après passage de la bille, qu'il y a un séchage en surface lorsque le film commence à présenter des arrachements, qu'il y a un séchage à coeur lorsque les arrachements disparaissent et, que le séchage est complet lorsque la bille ne laisse plus aucune marque sur le substrat.

Les résultats obtenus sont résumés dans le tableau 7 suivant.

**Tableau 7**

| | JB 041 | JB 042 |
|---|---|---|
| Film ne se referme plus | 20 min | 50 min |
| Début des arrachements | 30 min | 1 h |
| Fin des arrachements | 4h30 | 5h10 |
| Fin de trace de la bille* | > 11h | > 11h |

| | | |
|---|---|---|
| * : Le test a une durée de 11h, aussi il est possible que la trace de la bille soit toujours présente après cette durée sans que cela puisse être mis en évidence | | |

La phase initiale du séchage est rallongée de 30 min pour la peinture métallisée JB 042 et le retard pris lors de la 1^{ère} étape du séchage reste stable tout au long des différentes étapes. Ceci tend donc à montrer que les autres phases du séchage se font selon une cinétique similaire à celle de la peinture de l'art antérieur JB 041.

### Dureté du film de peinture

Les peintures métallisées JB 041 et JB 042 sont appliquées avec une épaisseur de film humide de 150 µm sur une plaque métallique. Les plaques ainsi produites sont soumises au test du pendule de Persoz afin d'évaluer leur dureté. Le pendule est posé à la surface du film de peinture et mis en oscillation. Plus la dureté du film sera élevée, plus l'amortissement des oscillations sera faible, plus le temps d'oscillation sera important. La dureté peut donc être « exprimée » en secondes, un temps plus long indiquant une dureté supérieure. Les résultats sont donnés dans le tableau 8 ci-dessous.

**Tableau 8**

| | J+1 | J+2 | J+7 | J+14 | J+30 |
|---|---|---|---|---|---|
| JB 041 | 47,3 | 55,7 | 75,3 | 126,3 | 217,3 |
| JB 042 | 47 | 91 | 144,3 | 171 | 209 |

La dureté initiale (1 jour après l'application) est identique pour les deux films de peintures. Ensuite, la dureté augmente de manière plus importante pour le film JB 042 selon l'invention. Mais après 30 jours, les deux films présentent une dureté similaire.

L'on peut ainsi conclure de l'ensemble des tests effectués, que le remplacement des pigments de l'art antérieur par des pigments selon la présente invention dans une composition de peinture du type alkyde-uréthane ne modifie pas les principaux paramètres caractéristiques de la peinture, i.e. brillance, stabilité, temps de séchage et dureté.

## Revendications

1. Procédé pour la préparation d'une composition de pigments métalliques, consistant à introduire des particules initiales de métal (Pi) dans un liquide support et à soumettre le mélange ainsi obtenu à un broyage, **caractérisé en ce que** le liquide support est constitué par un ester d'un acide gras R'-COOR dans lequel R' est un groupe aliphatique saturé ou insaturé ayant de 9 à 20 atomes de carbone, et R est un alkyle ayant de 1 à 8 atomes de carbone.

2. Procédé selon la revendication 1, **caractérisé en ce que** l'ester R'-COOR est choisi parmi ceux dans lesquels R' est un groupe saturé CₙH₂ₙ₊₁, n étant compris entre 9 et 20.

3. Procédé selon la revendication 2, **caractérisé en ce que** l'ester est le laurate de méthyle.

4. Procédé selon la revendication 1, **caractérisé en ce que** les particules de métal Pi sont choisies parmi les particules d'aluminium, de cuivre, de zinc, d'étain, d'or, d'argent, ou d'alliages de ces métaux, ainsi que parmi les particules d'acier inoxydable ou de bronze.

5. Procédé selon la revendication 1, **caractérisé en ce que** les particules métalliques Pi ont une dimension moyenne de 0,1 à 500 µm

6. Procédé selon la revendication 1, **caractérisé en ce que** le mélange soumis au broyage contient de 1 à 10 kg de liquide support pour 1 kg de particules Pi.

7. Procédé selon la revendication 1, **caractérisé en ce que** la durée du broyage est comprise entre 1 h et 20 h.

8. Procédé selon la revendication 1, **caractérisé en ce que**, à la fin de l'étape de broyage, la teneur en ester d'acide est ajustée à une valeur comprise entre 30 et 90% en masse de la masse totale.

9. Composition de pigments métalliques constituée par des particules métalliques Pf et un liquide, **caractérisée en ce que** le liquide est un ester d'un acide gras R'-COOR dans lequel R' est un groupe aliphatique saturé ou insaturé ayant de 9 à 20 atomes de carbone, et R est un alkyle ayant de 1 à 8 atomes de carbone.

10. Composition selon la revendication 9, **caractérisée en ce que** R est un méthyle et R' est un groupement alkyle saturé ayant de 9 à 17 atomes de carbone.

11. Composition selon la revendication 1, **caractérisé en ce que** la teneur en ester est comprise entre 30 et 90% en masse.

12. Composition selon la revendication 9, **caractérisée en ce que** les particules métalliques Pf sont des particules anisotropes ayant des dimensions moyennes inférieures ou égales à 500 µm et un facteur de forme, qui est le rapport de l'épaisseur moyenne au diamètre transversal moyen, compris entre 1/5 et 1/1000.

13. Composition selon la revendication 12, **caractérisée en ce que** les particules sont du type paillettes, avec un diamètre transversal moyen inférieur ou égal à 500 µm et une épaisseur moyenne inférieure ou égale à 3 µm.

14. Composition selon la revendication 9, **caractérisée en ce que** l'ester est le laurate de méthyle et les particules métalliques sont des paillettes d'aluminium.

15. Composition de peinture, **caractérisée en ce qu'**elle contient une composition de pigments métalliques selon la revendication 9.

16. Composition d'encre, **caractérisée en ce qu'**elle contient une composition de pigments métalliques selon la revendication 9.

17. Composition de matière plastique à aspect métallisé, **caractérisée en ce qu'**elle contient une composition de pigments métalliques selon la revendication 9.

18. Composition cosmétique à aspect métallisé, contenant une composition de pigments métalliques selon la revendication 9.

## Claims

1. A process for the preparation of a composition formed of metal pigments which consists in introducing starting metal particles (SP) into a liquid vehicle and in subjecting the mixture thus obtained to milling, **characterized in that** the liquid vehicle is composed of an ester of a fatty acid R'-COOR in which R' is a saturated or unsaturated aliphatic group having from 9 to 20 carbon atoms and R is an alkyl having from 1 to 8 carbon atoms.

2. The process as claimed in claim 1, **characterized in that** the ester R'-COOR is chosen from those in which R' is a saturated CₙH₂ₙ₊₁ group, n being between 9 and 20.

3. The process as claimed in claim 2, **characterized in that** the ester is methyl laurate.

4. The process as claimed in claim 1, **characterized in that** the metal particles SP are chosen from particles of aluminum, of copper, of zinc, of tin, of gold, of silver or of alloys of these metals, as well as from particles formed of stainless steel or of bronze.

5. The process as claimed in claim 1, **characterized in that** the metal particles SP have a mean dimension of 0.1 to 500 µm.

6. The process as claimed in claim 1, **characterized in that** the mixture subjected to milling comprises from 1 to 10 kg of liquid vehicle per 1 kg of SP particles.

7. The process as claimed in claim 1, **characterized in that** the duration of the milling is between 1 h and 20 h.

8. The process as claimed in claim 1, **characterized in that**, at the end of the milling stage, the content of acid ester is adjusted to a value of between 30 and 90% by weight of the total weight.

9. A composition formed of metal pigments which is composed of metal particles FP and a liquid, **characterized in that** the liquid is an ester of a fatty acid R'-COOR in which R' is a saturated or unsaturated aliphatic group having from 9 to 20 carbon atoms and R is an alkyl having from 1 to 8 carbon atoms.

10. The composition as claimed in claim 9, **characterized in that** R is a methyl and R' is a saturated alkyl group having from 9 to 17 carbon atoms.

11. The composition as claimed in claim 1, **characterized in that** the ester content is between 30 and 90% by weight.

12. The composition as claimed in claim 9, **characterized in that** the metal particles FP are anisotropic particles having mean dimensions of less than or equal to 500 µm and a form factor, which is the ratio of the mean thickness to the mean transverse diameter, of between 1/5 and 1/1000.

13. The composition as claimed in claim 12, **characterized in that** the particles are of the glitter type with a mean transverse diameter of less than or equal to 500 µm and a mean thickness of less than or equal to 3 µm.

14. The composition as claimed in claim 9, **characterized in that** the ester is methyl laurate and the metal particles are aluminum glitter.

15. A paint composition, **characterized in that** it comprises a composition formed of metal pigments as claimed in claim 9.

16. An ink composition, **characterized in that** it comprises a composition formed of metal pigments as claimed in claim 9.

17. A composition formed of plastic with a metallic appearance, **characterized in that** it comprises a composition formed of metal pigments as claimed in claim 9.

18. A cosmetic composition with a metallic appearance, comprising a composition formed of metal pigments as claimed in claim 9.

## Patentansprüche

1. Verfahren zur Herstellung einer Metallpigmentzusammensetzung, das darin besteht, Metallausgangsteilchen (Pi) in eine Trägerflüssigkeit einzuführen und das so erhaltene Gemisch zu zerkleinern, **dadurch gekennzeichnet, dass** die Trägerflüssigkeit aus einem Fettsäureester R'-COOR besteht, worin R' eine gesättigte oder ungesättigte aliphatische Gruppe mit 9 bis 20 Kohlenstoffatomen ist und R ein Alkyl mit 1 bis 8 Kohlenstoffatomen ist.

2. Verfahren nach Anspruch 1, **dadurch gekennzeichnet, dass** der Ester R'-COOR aus jenen ausgewählt ist, in denen R' eine gesättigte Gruppe der Formel CₙH₂ₙ₊₁ ist, worin n eine Zahl zwischen 9 und 20 ist.

3. Verfahren nach Anspruch 2, **dadurch gekennzeichnet, dass** der Ester Methyllaurat ist.

4. Verfahren nach Anspruch 1, **dadurch gekennzeichnet, dass** die Metallteilchen Pi aus Aluminium-, Kupfer-, Zink-, Zinn-, Gold-, Silberteilchen und Teilchen aus Legierungen dieser Metalle sowie aus Edelstahl- und Bronzeteilchen ausgewählt sind.

5. Verfahren nach Anspruch 1, **dadurch gekennzeichnet, dass** die Metallteilchen Pi eine mittlere Größe von 0,1 bis 500 µm aufweisen.

6. Verfahren nach Anspruch 1, **dadurch gekennzeichnet, dass** das dem Zerkleinern unterzogene Gemisch 1 bis 10 kg Trägerflüssigkeit pro 1 kg Teilchen Pi enthält.

7. Verfahren nach Anspruch 1, **dadurch gekennzeichnet, dass** die Dauer des Zerkleinerns zwischen 1 h und 20 h beträgt.

8. Verfahren nach Anspruch 1, **dadurch gekennzeichnet, dass** am Ende des Zerkleinerungsschritts der Säureestergehalt auf einen Wert zwischen 30 und 90 Gew.-% des Gesamtgewichts eingestellt wird.

9. Metallpigmentzusammensetzung aus Metallteilchen Pf und einer Flüssigkeit, **dadurch gekennzeichnet, dass** die Flüssigkeit ein Fettsäureester R'-COOR ist, worin R' eine gesättigte oder ungesättigte aliphatische Gruppe mit 9 bis 20 Kohlenstoffatomen und R ein Alkyl mit 1 bis 8 Kohlenstoffatomen ist.

10. Zusammensetzung nach Anspruch 9, **dadurch gekennzeichnet, dass** R ein Methyl und R' eine gesättigte Alkylgruppe mit 9 bis 17 Kohlenstoffatomen ist.

11. Zusammensetzung nach Anspruch 1, **dadurch gekennzeichnet, dass** der Estergehalt zwischen 30 und 90 Gew.-% beträgt.

12. Zusammensetzung nach Anspruch 9, **dadurch gekennzeichnet, dass** die Metallteilchen Pf anisotrope Teilchen sind, die eine mittlere Größe von 500 µm oder weniger und einen Formfaktor, der das Verhältnis zwischen der mittleren Dicke und dem mittleren Querrdurchmesser ist, zwischen 1/5 und 1/1000 aufweisen.

13. Zusammensetzung nach Anspruch 12, **dadurch gekennzeichnet, dass** die Teilchen vom Plättchen-Typ mit einem mittleren Querdurchmesser von 500 µm oder weniger und einer mittleren Dicke von 3 µm oder weniger sind.

14. Zusammensetzung nach Anspruch 9, **dadurch gekennzeichnet, dass** der Ester Methyllaurat ist und die Metallteilchen Aluminiumplättchen sind.

15. Anstrichfarbenzusammensetzung, **dadurch gekennzeichnet, dass** diese eine Metallpigmentzusammensetzung nach Anspruch 9 enthält.

16. Tintenzusammensetzung, **dadurch gekennzeichnet, dass** diese eine Metallpigmentzusammensetzung nach Anspruch 9 enthält.

17. Kunststoffmaterialzusammensetzung mit metallisiertem Erscheinungsbild, **dadurch gekennzeichnet, dass** diese eine Metallpigmentzusammensetzung nach Anspruch 9 enthält.

18. Kosmetische Zusammensetzung mit metallisiertem Erscheinungsbild, die eine Metallpigmentzusammensetzung nach Anspruch 9 enthält.
